# EUROPÄISCHE PATENTSCHRIFT

(11) **EP 0 593 948 B1**
(45) Veröffentlichungstag und Bekanntmachung des Hinweises auf die Patenterteilung: **27.05.1998**
(21) Anmeldenummer: 93115605.3
(22) Anmeldetag: 28.09.1993
(51) Int. Cl.: A61M 25/04

(54) **Stent-Set**
Stent-set
Cathéter endurétral et dispositif d'application

(30) Priorität: 09.10.1992 DE 9213656 U
(43) Veröffentlichungstag der Anmeldung: 27.04.1994
(73) Patentinhaber: ANGIOMED GmbH & Co. Medizintechnik KG, 76227 Karlsruhe (DE)
(72) Erfinder: Lindenberg, Josef, D-76227 Karlsruhe (DE); Schnepp-Pesch, Wolfram, D-76185 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- WO-A-91/10467
- US-A- 4 973 301

## Beschreibung

Die Erfindung betrifft ein Stent-Set nach dem Oberbegriff des Anspruchs 1.

Intraurethral-Katheter dienen zur Behandlung infravesicaler Verschlüsse , wie zum Beispiel Blasenhalsadenomen, Harnröhrenverengungen oder Prostatakarzinomen, und stellen eine vorteilhafte Alternative zu einem einzuführenden, sich durch die gesamte Harnröhre erstreckenden und aus dieser in die Blase ragenden Katheter mit abgebogenem Ende dar, wenn sich der Patient auf einer Warteliste befindet oder aber sich einer Operation nicht unterziehen will oder kann. Das Einführen des Intraurethral-Katheters erfolgt dabei ambulant unter lokaler Anästhesie. Derartige zylinderförmige Intraurethral-Katheter sind im Endbereich geschlitzt und weisen deshalb mehrere gleichförmige, über den Umfang gleichwertige, zu einem Trichter oder einer Krone geformte aufspreizbare Mantelabschnitte auf, durch die der Harn aus der Blase zur Entleerung derselben in den innen ausgebildeten Abflußkanal gelangt. Aufgrund dieser Ausbildung kann es jedoch zu Inkrustationen in der Blase kommen, was nachfolgend zu einer Entzündung führt. Deshalb müssen derartige Intraurethral-Katheter immer wieder ersetzt werden.

Ein gattungsgemäßes Stent-Set ist aus der US-PS 4,973,301 bekannt. Dieses Stent-Set weist u.a. einen Intraurethral-Katheter, eine rohrförmige Einführschiene sowie einen Schieber auf. Der Intraurethral-Katheter wird durch einen zylindrischen Grundkörper gebildet, an dessen Endbereichen Körbchen ausgebildet sind. Das proximale Ende des Katheters ist mit einem zylindrisch ausgebildeten Ende versehen, welches zur Ausbildung eines Trichters auch geschlitzt sein kann. Ist die Einführschiene bei diesem Stent-Set durch die Urethra eingeschoben, so wird der innerhalb einer Hülse befindliche Katheter zunächst mittels des an seinem distalen Ende angreifenden Schiebers in die Einführschiene und nachfolgend aus dieser herausgeschoben, so daß sich das proximale Körbchen in der Blase befindet.

Ein wesentlicher Nachteil beim Stande der Technik besteht auch darin, daß der Schieber zum Herausschieben des Katheters aus der Einführschiene am rückwärtigen Ende des Katheters angreifen muß, wodurch der Katheter gestaucht und damit seine Wandung insbesondere im Bereich der Körbchen stärker gegen die Innenwandseite der Einführschiene gedrückt wird und so letztendlich das Herausschieben des Katheters sehr erschwert wird.

Die WO 91/10467 zeigt einen zylindrischen Katheter, der an seinem proximalen Ende mit einer abgerundeten Spitze und seitlichen Öffnungen versehen ist.

Der Erfindung liegt daher die Aufgabe zugrunde, ein gattungsgemäßes Stent-Set mit Intraurethral-Katheter, Einführschiene und Schieber zu schaffen, welches unter Vermeidung der vorgenannten Nachteile eine sichere Einführung mit weniger Belastung für den Patienten ermöglicht, wobei der Intraurethral-Katheter permanent im Körperinneren verbleiben kann.

Erfindungsgemäß wird die genannte Aufgabe bei einem Stent-Set der eingangs genannten Art durch die Merkmale des kennzeichnenden Teils des Anspruchs 1 gelöst.

Die Erfindung hat damit den wesentlichen Vorteil, daß zum Vorschieben des Intraurethral-Katheters mittels des Schiebers an seiner vorderen, geschlossenen oder mit einer Öffnung kleiner als der Querschnitt des Schiebers versehenen Spitze angegriffen wird, und nicht am rückwärtigen Ende, wie dies beim Stand der Technik der Fall ist. Durch dieses erfindungsgemäße Angreifen wird erreicht, daß der Intraurethral-Katheter durch den Schieber "gezogen" wird; er wird hierdurch beim Vorbewegen gestreckt, vergrößert sich etwas und kann leicht ohne große Reibung aus dem vorzugsweise abgebogenen vorderen Ende seiner Einführschiene herausbewegt werden. Demgegenüber wird der Intraurethral-Katheter beim Stand der Technik gestaucht, wenn er von seinem hinteren Ende aus der Schiene herausgeschoben wird. Durch das Stauchen weitet sich der Intraurethral-Katheter leicht auf, wodurch die Reibung zwischen ihm und der Einführschiene vergrößert und damit das Herausschieben erschwert ist.

In bevorzugter Weiterbildung ist vorgesehen, daß der Intraurethral-Katheter an seinem proximalen Ende weitere Öffnungen aufweist, welche eine Verbindung zum Abflußkanal innerhalb des Intraurethral-Katheters darstellen und durch welche während des Einführvorganges sofort Harn in den Abflußkanal des Intraurethral-Katheter eintritt und am distalen Ende der innen hohl ausgebildeten Einführschiene austritt, so daß von außen ersichtlich ist, daß die Harnblase erreicht ist. Dies ist deshalb möglich, da der in die Einführschiene eingeschobene Intraurethral-Katheter aufgrund eines innerhalb der Einführschiene befindlichen Führungsteils nicht vollständig eingeschoben werden kann und daher seine abgerundete Spitze mit den Öffnungen am proximalen Ende aus der Einführschiene herausragt.

Überdies werden die flachen Kanten der Einführschiene durch die abgerundete Spitze des Intraurethral-Katheters abgemildert, so daß es beim Einführen nicht zu Verletzungen oder Belastungen des Patienten kommt. Weiterbildungen sehen vor, daß der Intraurethral-Katheter bevorzugt aus hochflexiblem Material, z.B. Polyethylen oder Polyurethan, besteht. Aufgrund dieser Ausgestaltung läßt sich der Intraurethral-Katheter leicht in die Einführschiene einziehen, wobei die radial aufgeweiteten Körbchen dann leicht durch die Einführschiene zusammengepreßt werden können. Nach dem Heraustreten aus der Einführschiene, wenn diese zurückgezogen wird, können sich die Körbchen aufgrund der Entlastung selbständig wiederum radial aufstellen. Da die Gefäße überdies nicht immer gestreckt sind und Krümmungen aufweisen, wird durch das verwendete Material die notwendige Biegsamkeit gewährleistet.

In weiterer Ausgestaltung ist vorgesehen, daß die Einführschiene am vorderen Ende leicht gegen die Erstreckungsrichtung ihres Hauptkörpers geneigt oder abgewinkelt ist und daß ein abgewinkelter Endbereich eine zylindrische Form aufweist, wobei die Länge des Endbereichs zwischen 1 und 3 cm liegt. Der Endbereich ist vorzugsweise im Bereich von 10° bis 50° zur Haupterstreckungsrichtung des Hauptkörpers der Einführschiene abgewinkelt, weist also zu diesem einen stumpfen Winkel zwischen 130° und 170° auf. Dabei ist ein Krümmungsradius nicht über die gesamte Länge gegeben, sondern nur in einem sehr kurzen Krümmungsbereich, über den die Abbiegung um den gewünschten Winkel erfolgt und an den sich der gestreckte Endbereich anschließt, der gegenüber dem Hauptkörper unter dem gewünschten Winkel ausgerichtet ist. Die Einführschiene besteht dabei aus einem relativ steifen Material, so daß der abgewinkelte Endbereich durch den aus hochflexiblem aber axial relativ steifem Material bestehenden Schieber und den aus ebenfalls hochflexiblem Material bestehenden Intraurethral-Katheter nicht aufgestellt werden kann, sondern leicht abgebogen bleibt, wenn Schieber und Intraurethral-Katheter sich innerhalb der Einführschiene befinden. Der Endbereich kann allerdings beim Einführvorgang gesteuert werden, indem die Einführschiene leicht verdreht wird. Damit kann die auf das Führungsteil rutschfest aufgeschobene Einführschiene sicher durch die Abbiegungen aufweisenden Gänge geführt werden.

Insgesamt wird durch die Erfindung ein Stent-Set geschaffen, welches nicht nur aufgrund seines abgewinkelten Endbereichs durch Verdrehen der Einführschiene steuerbar ist, sondern durch welches auch leicht und sicher ein Intraurethral-Katheter in den vorgesehenen Bereich eingeführt werden kann, wobei dieser dann aufgrund seiner Ausgestaltung permanent im Körperinneren verbleiben kann, ohne daß es aufgrund von Inkrustationen zu Infektionen kommt.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnungen im einzelnen erläutert ist. Dabei zeigt bzw. zeigen:
- Figur 1: die bevorzugte Ausgestaltung des erfindungsgemäßen Stent-Sets
- Figur 2: die bevorzugte Ausgestaltung eines zum erfindungsgemäßen Stent-Sets gehörenden Intraurethral-Katheters
- Figur 3: die bevorzugte Ausgestaltung des zum erfindungsgemäßen Stent-Sets gehörenden Schiebers

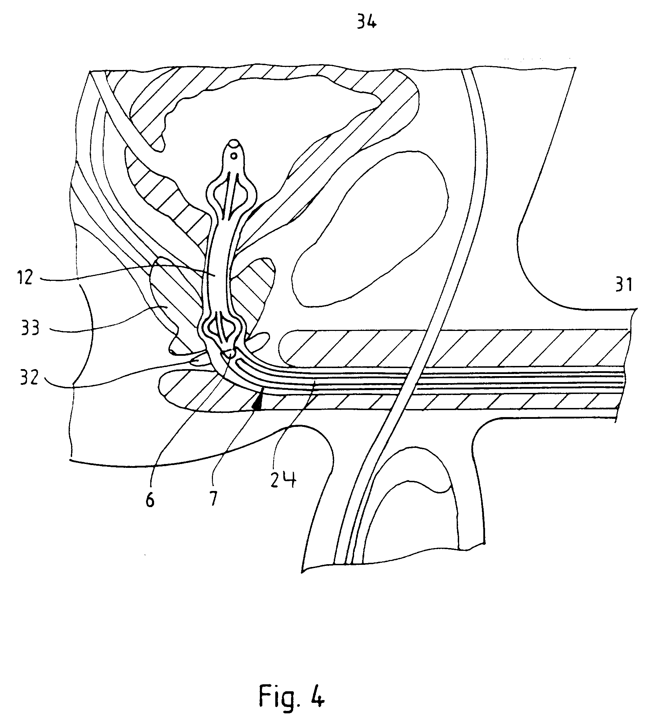

- Figur 4: eine Darstellung zur vorderen Krümmung der Einführschiene.

Das erfindungsgemäße Stent-Set 1 weist eine hohle Einführschiene 2 auf. Die Einführschiene 2 weist an ihrem vorderen proximalen - d.h. nach Einführen zum Körperzentrum zeigenden - Ende einen abgewinkelten Krümmungsbereich 7 auf, an den sich wiederum ein kurzer, gestreckter Endbereich 6 anschließt. Der Endbereich 6 schließt zum Hauptkörper der Einführschiene 2 einen stumpfen Winkel von 130° bis 170° ein. In der Einführschiene 2 befindet sich ein in den Körper des Patienten einzubringender Intraurethral-Katheter 12. Das Stent-Set 1 kann weiter innerhalb der Einführschiene 2 ein Führungsteil 4 - mit rückwärtigem Griffteil 3- zum Einführen eines Schiebers 24 in den Intraurethral-Katheter 12 aufweisen. Das Führungsteil 4 dient dabei als Anschlag oder Einschubbegrenzung für den Intraurethral-Katheter 12, so daß dieser beim Vorschieben der Einführschiene 2 nicht weiter in diese zurückgeschoben wird. Das Führungsteil 4 weist eine derartige Länge auf, daß bei auf das Führungsteil 4 vollständig aufgeschobener Einführschiene 2 und in die Einführschiene 2 eingeschobenem Intraurethral-Katheter 12 dieser mit seinem hinteren Ende 13 an das vordere Ende 14 des Führungsteils 4 anstößt, während die abgerundete Spitze 15 des Intraurethral-Katheters 12 aus dem proximalen Ende der Einführschiene 2 herausragt.

Der Schieber 24 stößt, vollständig in Einführschiene 2, gegebenenfalls Führungsteil 4 und Intraurethral-Katheter 12 eingeführt, von innen an die abgerundete Spitze 15 des Intraurethral-Katheters 12.

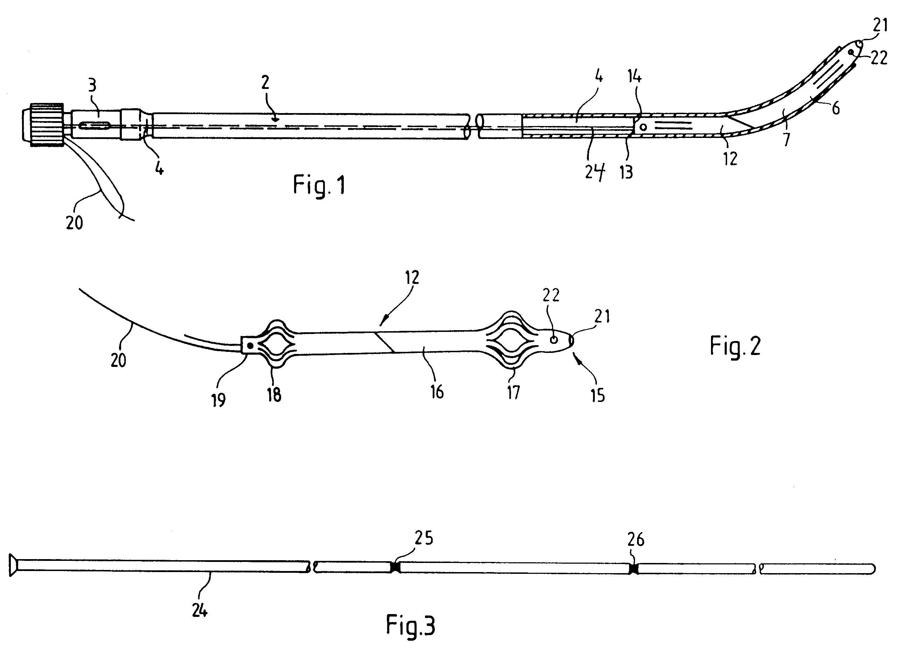

Der in Figur 2 dargestellte hochelastische, bevorzugt aus Polyethylen bzw. Polyurethan bestehende Intraurethral-Katheter 12 weist einen zylindrischen Grundkörper mit jeweils einem Körbchen 18,17 am proximalen bzw. distalen Ende und einen länglichen Teil 16 festgelegter Länge zwischen den Körbchen 18,17 auf, wobei diese einstückig mit dem länglichen Teil 16 ausgebildet sind und sich oberhalb des Körbchens 17 am proximalen Ende eine einstückig mit dem Körbchen 17 verbundene, abgerundete Spitze 15 sowie sich unterhalb des Körbchens 18 an seinem der abgerundeten Spitze 15 abgewandten Seite ein mit diesem einstückig verbundenes, zylindrisches Endteil 13 befindet. Die Körbchen 18, 17 sind durch Längsschlitze im Grundkörper des Intraurethral-Katheters 12, Stauchen des Intraurethral-Katheters 12, wodurch sich die verbleibenden Rippen in diesem Bereich nach außen aufstellen, und Fixieren dieser Stellung durch Wärmebehandlung gebildet. Der Intraurethral-Katheter 12 kann auf der distalen Hälfte eine andere Farbe aufweisen als auf der proximalen Hälfte.

Das Endteil 13 weist eine obere Stirnseite auf sowie eine seitliche Öffnung 19 zur Aufnahme eines Fadens 20. Beim durch die Öffnung 19 geführten Faden 20 handelt es sich dabei bevorzugt um einen Doppelfaden, wobei die Enden auf ihrem dem Intraurethral-Katheter 12 abgewandten Ende miteinander verknotet sind. Der verknotete Doppelfaden 20 weist zumindest eine Länge auf, die das eineinhalbfache der Länge von Einführschiene 2 und Führungsteil 4 (aufeinander geschoben) beträgt. Mittels des Doppelfadens 20 kann der Intraurethral-Katheter 12 später wieder entfernt werden.

Das Körbchen 17 am proximalen Ende ist größer ausgebildet als das Körbchen 18 am distalen Ende.

Der bevorzugt aus einem hochflexiblen aber axial relativ steifem Material bestehende Schieber 24 (Figur 3) weist zwei Marken 25,26 in Form von Einkerbungen auf, welche ein unterschiedliches Reflektionsvermögen besitzen.

Der Intraurethral-Katheter 12 weist an seinem vorderen Ende Öffnungen 21,22 auf, durch die nach Hindurchtreten des Intraurethral-Katheters 12 in die Harnblase Flüssigkeit in die Einführschiene 2 bzw. das Führungsteil 4 ein- und am rückwärtigen Ende desselben austritt, wodurch die Position des Intraurethral-Katheters 12 angezeigt wird.

Der Intraurethral-Katheter 12 wird mittels der Einführschiene 2 in an sich bekannter Weise durch die Urethra 31 eingeführt. Die Urethra 31 weist im Übergangsbereich zum Sphinkter 32 und zur Prostata 33 eine Krümmung auf, an die der Krümmungsbereich 7 der Einführschiene 2 angepaßt ist. Ist die Einführschiene 2 durch die Urethra eingeschoben, wird der Schieber 24 vom distalen Ende der Einführschiene 2 her bis in die Spitze 15 des Intraurethral-Katheters 12 geschoben. Dieser Anstoß ist von außen dadurch ersichtlich, daß die erste Marke 26 gerade noch aus dem distalen Ende der Einführschiene 2 bzw. des Führungsteils 4 herausragt. Der Intraurethral-Katheter 12 wird durch den Schieber 24 aus der Einführschiene 2 herausgeschoben, so daß sich das erste - vordere - Körbchen 17 in der Blase 34 befindet und das hintere Körbchen 18 in der Harnröhre 31 im Bereich der Prostata 33, so daß der Sphinkter 32 - nach Entfernen der Einführschiene 2 - nicht beeinträchtigt wird. Ist das erste Körbchen 17 vollständig aus der Einführschiene 2 herausgeschoben, so wird dies durch die zweite Marke 25 angezeigt. Durch die Öffnungen 21, 22 tritt bei Einführung bei Erreichen der Harnblase 34 sofort Harn aus, so daß von außen ersichtlich ist, daß diese erreicht ist. Nach dem Heraustreten aus der Einführschiene 2 weiten sich die Körbchen 17,18 radial auf, so daß durch die Öffnungen zwischen den Mantelabschnitten des Körbchens 17 Harn eintreten und durch die Öffnung des Körbchens 18 austreten kann. Nach erfolgter Einführung werden Schieber 24, Einführschiene 2 und gegebenenfalls Führungsteil 4 zurückgezogen und aus der Harnröhre 31 entfernt. Der Doppelfaden 20 kann dabei am Intraurethral-Katheter 12 verbleiben, um diesen zu entfernen oder aber er wird bei permanentem Verbleib des Intraurethral-Katheters 12 im Körperinneren abgeschnitten und entfernt, da er ansonsten eine Infektionsquelle darstellen kann.

Der durch das erfindungsgemäße Stent-Set 1 eingeführte Intraurethral-Katheter 12 kann nun permanent im Körperinneren verbleiben oder aber entfernt werden, wenn er - z.B. aufgrund einer durchgeführten Operation - nicht mehr benötigt wird.

## Patentansprüche

1. Stent-Set zum Offenhalten von Verengungen der Harnröhre, insbesondere im Bereich des Blasenhalses zur Blasendrainage, einen Intraurethral-Katheter (12) mit einem über einen großen Teil zylindrischen Grundkörper mit Körbchen (17, 18) an den Enden, eine Einführschiene (2) und einen Schieber (24) aufweisend, wobei eine Öffnung (21) an der Stirnseite des Intraurethral-Katheters (12) ausgebildet ist,
**dadurch gekennzeichnet**,
daß der Intraurethral-Katheter (12) an seinem proximalen Ende eine abgerundete Spitze (15) aufweist, in der die Öffnung (21) ausgebildet ist, und daß der Durchmesser des an der abgerundeten Spitze (15) des Intraurethral-Katheters (12) angreifenden Schiebers (24) größer als der der stirnseitigen Öffnung (21) ist.

2. Stent-Set nach Anspruch 1, dadurch gekennzeichnet, daß der Intraurethral-Katheter (12) seitlich an seinem proximalen Ende Öffnungen (22) aufweist.

3. Stent-Set nach Anspruch 1 oder 2, dadurch gekennzeichnet, daß ein erstes Körbchen (17) am vorderen proximalen Ende des Intraurethral-Katheters (12) unterhalb der abgerundeten Spitze (15) größer ausgebildet ist als ein zweites Körbchen (18) am rückwärtigen distalen Ende des Intraurethral-Katheters (12) oberhalb eines zylindrischen Endteils (13).

4. Stent-Set nach einem der Ansprüche 1 bis 3, dadurch gekennzeichnet, daß zwischen den Körbchen (17,18) ein zylindrisch ausgebildeter, länglicher Tel (16) ausgebildet ist.

5. Stent-Set nach Anspruch 3 oder 4, dadurch gekennzeichnet, daß die Körbchen (17,18), der längliche Teil (16) zwischen ihnen sowie das Endteil (13) und die abgerundete Spitze (15) des Intraurethral-Katheters (12) einstückig miteinander verbunden sind.

6. Stent-Set nach einem der Ansprüche 1 bis 5, dadurch gekennzeichnet, daß der Intraurethral-Katheter (12) an seinem der abgerundeten Spitze (15) abgewandten zylindrischen Endteil (13) eine Öffnung (19) zur Aufnahme eines Fadens (20) aufweist.

7. Stent-Set nach einem der Ansprüche 1 bis 6, dadurch gekennzeichnet, daß der Intraurethral-Katheter an der Stirnseite seines der abgerundeten Spitze (15) abgewandten zylindrischen Endteils (13) mit flachen Kanten versehen ist.

8. Stent-Set nach einem der vorangehenden Ansprüche, dadurch gekennzeichnet, daß die Einführschiene (2) am vorderen Ende leicht gegen die Erstreckungsrichtung ihres Hauptkörpers geneigt oder abgewinkelt ist.

9. Stent-Set nach Anspruch 8, dadurch gekennzeichnet, daß ein vorderer Endbereich (6) der Einführschiene (2) um 10° bis 50° von der Erstreckungsrichtung des Hauptkörpers (9) der Einführschiene (2) abgewinkelt ist, also zum Hauptkörper einen Winkel zwischen 130° und 170° einschließt.

10. Stent-Set nach Anspruch 8 oder 9, dadurch gekennzeichnet, daß sich an den Hauptkörper der Einführschiene (2) ein kurzer Krümmungsbereich (7) und an diesen wiederum der gestreckte kurze Endbereich (6) anschließt.

11. Stent-Set nach Anspruch 10, dadurch gekennzeichnet, daß der abgewinkelte Endbereich (6) zylindrisch ausgebildet ist.

12. Stent-Set nach einem der vorangehenden Ansprüche, dadurch gekennzeichnet, daß die Einführschiene (2) einen Innendurchmesser aufweist, der dem Außendurchmesser eines Führungsteils (4) für den Schieber (24) entspricht.

13. Stent-Set nach einem der vorangehenden Ansprüche, dadurch gekennzeichnet, daß der in die Einführschiene (2) eingeschobene Intraurethral-Katheter (12) mit seinem distalen Ende (13) gegen das proximale Ende (14) des Führungsteils (4) stößt und seine abgerundete Spitze (15) aus dem vorderen Ende der Einführschiene (2) herausragt.

14. Stent-Set nach Anspruch 13, dadurch gekennzeichnet, daß der Intraurethral-Katheter (12) einen dem Innendurchmesser der Einführschiene (2) entsprechenden Außendurchmesser aufweist.

15. Stent-Set nach einem der Ansprüche 1 bis 14, dadurch gekennzeichnet, daß der Schieber (24) zwei Marken (25, 26) in Form einer Einkerbung mit einem gegenüber dem Rest des Schiebers (24) geänderten Reflektionsvermögen aufweist.

16. Stent-Set nach Anspruch 1 oder 15, dadurch gekennzeichnet, daß der Schieber (24) mindestens die eineinhalbfache Länge der Einführschiene (2) aufweist.

17. Stent-Set nach einem der vorangehenden Ansprüche, dadurch gekennzeichnet, daß der Schieber (24) einen gegenüber den Innendurchmessern des Intraurethral-Katheters (12) und gegebenenfalls des Führungsteils (4) geringeren Außendurchmesser aufweist.

18. Stent-Set nach einem vorangehenden Anspruch, dadurch gekennzeichnet, daß die Einführschiene (2) aus relativ steifem Material besteht.

19. Stent-Set nach einem der vorangehenden Ansprüche, dadurch gekennzeichnet, daß der Intraurethral-Katheter (12) aus hochflexiblem Material besteht.

20. Stent-Set nach einem der vorangehenden Ansprüche, dadurch gekennzeichnet, daß der Schieber (24) aus hochflexiblem aber axial relativ steifem Material besteht.

21. Stent-Set nach Anspruch 19 oder 20, dadurch gekennzeichnet, daß es sich bei dem hochflexiblen Material um Polyethylen handelt.

22. Stent-Set nach Anspruch 19 und 20, dadurch gekennzeichnet, daß es sich bei dem hochflexiblen Material um Polyurethan handelt.

## Claims

1. Stent set for keeping open stenoses of the urethra, particularly in the area of the bladder neck for bladder drainage, an interurethral catheter (12) with a largely cylindrical body having small baskets (17, 18) at the ends, an insertion rail (2) and a slider (24), an opening (21) being formed on the front face of the intraurethral catheter (12), characterized in that the intraurethral catheter (12) has at its proximal end a rounded tip (15) in which is formed an opening (21), and that the diameter of the slider (24) engaging on the rounded tip (15) of the intraurethral catheter (12) is larger than that of the front opening (21).

2. Stent set according to claim 1, characterized in that the intraurethral catheter (12) is laterally provided at its proximal end with openings (22).

3. Stent set according to claim 1 or 2, characterized in that a first basket (17) at the front, proximal end of the intraurethral catheter (12) below the rounded tip (15) is larger than a second basket (18) at the rear, distal end of the intraurethral catheter (12) above a cylindrical end part (13).

4. Stent set according to one of the claims 1 to 3, characterized in that a cylindrical, elongated part (16) is formed between the baskets (17, 18).

5. Stent set according to claim 3 or 4, characterized in that the baskets (17, 18), the elongated part (16) between them, as well as the end part (13) and the rounded tip (15) of the intraurethral catheter (12) are interconnected in one piece.

6. Stent set according to one of the claims 1 to 5, characterized in that on the cylindrical end part (13) of the intraurethral catheter (12) remote from the rounded tip (15) is provided an opening (19) for receiving a thread (20).

7. Stent set according to one of the claims 1 to 6, characterized in that on the front of the cylindrical end part (13) of the intraurethral catheter remote from the rounded tip (15) are provided flat edges.

8. Stent set according to one of the preceding claims, characterized in that the front end of the insertion rail (2) is slightly inclined or bent against the extension direction of its main body.

9. Stent set according to claim 8, characterized in that a front end region (6) of the insertion rail (2) is bent by 10 to 50° from the extension direction of the main body (9) of the insertion rail (2), i.e. forms with the main body an angle between 130 and 170°.

10. Stent set according to claim 8 or 9, characterized in that to the main body of the insertion rail (2) is connected a short curvature area (7) and to the latter the stretched, short end region (6).

11. Stent set according to claim 10, characterized in that the bent end region (6) has a cylindrical construction.

12. Stent set according to one of the preceding claims, characterized in that the insertion rail (2) has an internal diameter corresponding to the external diameter of a guide part (4) for the slider (24).

13. Stent set according to one of the preceding claims, characterized in that the distal end (13) of the intraurethral catheter (12) slid into the insertion rail (2) strikes against the proximal end (14) of the guide part (4) and its rounded tip (15) projects out of the front end of the insertion rail (2).

14. Stent set according to claim 13, characterized in that the external diameter of the intraurethral catheter (12) corresponds to the internal diameter of the insertion rail (2).

15. Stent set according to one of the claims 1 to 14, characterized in that the slider (24) has two marks (25, 26) in the form of an indentation with a reflecting power modified compared with the remainder of the slider (24).

16. Stent set according to claim 1 or 15, characterized in that the slider (24) is at least one and a half times as long as the insertion rail (2).

17. Stent set according to one of the preceding claims, characterized in that the slider (24) has a smaller external diameter than the internal diameter of the intraurethral catheter (12) and optionally the guide part (4).

18. Stent set according to one of the preceding claims, characterized in that the insertion rail (2) is made from relatively stiff material.

19. Stent set according to one of the preceding claims, characterized in that the intraurethral catheter (12) is made from highly flexible material.

20. Stent set according to one of the preceding claims, characterized in that the slider (24) is made from highly flexible, but axially relatively stiff material.

21. Stent set according to claim 19 or 20, characterized in that the highly flexible material is polyethylene.

22. Stent set according to claim 19 and 20, characterized in that the highly flexible material is polyurethane.

## Revendications

1. Ensemble extenseur pour l'expansion de rétrécissements de l'urètre, en particulier dans la zone du col de la vessie pour le drainage de la vessie, comprenant un cathéter endurétral (12) présentant un corps de base cylindrique sur sa majeure partie muni de bulbes (17,18) à ses extrémités, un guide d'introduction (2) et un coulisseau (24), une ouverture (21) étant ménagée à l'extrémité frontale du cathéter endurétral (12), caractérisé en ce que le cathéter endurétral (12) présente à son extrémité proximale une pointe arrondie (15) dans laquelle est conformée l'ouverture, et en ce que le diamètre du coulisseau (24) venant en contact avec la pointe arrondie (15) du cathéter endurétral (12) est supérieur à celui de l'ouverture frontale (21).

2. Ensemble extenseur selon la revendication 1, caractérisé en ce que le cathéter endurétral (12) présente latéralement à son extrémité proximale des ouvertures (22).

3. Ensemble extenseur selon la revendication 1 ou 2, caractérisé en ce qu'un premier bulbe (17) conformé à l'extrémité proximale avant du cathéter endurétral (12) en-dessous de la pointe arrondie (15) est plus grand qu'un deuxième bulbe (18) conformé à l'extrémité arrière distale du cathéter endurétral (12) au-dessus d'un segment d'extrémité cylindrique (13).

4. Ensemble extenseur selon l'une quelconque des revendications 1 à 3, caractérisé en ce qu'un segment longitudinal (16) de conformation cylindrique s'étend entre les bulbes (17,18)..

5. Ensemble extenseur selon la revendication 3 ou 4, caractérisé en ce que les bulbes (17,18), le segment longitudinal (16) situé entre eux ainsi que le segment d'extrémité (13) et les pointes arrondies (15) du cathéter endurétral sont d'une seule pièce.

6. Ensemble extenseur selon l'une quelconque des revendications 1 à 5, caractérisé en ce que le cathéter endurétral (12) présente à son segment cylindrique d'extrémité (13) opposé à l'extrémité arrondie (15) une ouverture (19) pour recevoir un fil (20).

7. Ensemble extenseur selon l'une quelconque des revendications 1 à 6, caractérisé en ce que le cathéter endurétral présente à l'extrémité frontale de son segment d'extrémité cylindrique (13) opposé à la pointe arrondie (15) des chants plats.

8. Ensemble extenseur selon l'une quelconque des revendications précédentes, caractérisé en ce que le guide d'introduction (2) est légèrement courbé ou plié dans le sens contraire de la direction d'extension de son corps principal.

9. Ensemble extenseur selon la revendication 8, caractérisé en ce qu'une zone d'extrémité avant (6) du guide d'introduction (2) est pliée selon un angle de 10 à 50° par rapport à la direction d'extension du corps principal (9) du guide d'introduction (2), formant ainsi par rapport au corps principal un angle de 130 à 170°.

10. Ensemble extenseur selon la revendication 8 ou 9, caractérisé en ce qu'une courte zone de courbure (7) prolonge le corps principal du guide d'introduction (2) lui-même prolongé par la courte zone d'extrémité (6) droite.

11. Ensemble extenseur selon la revendication 10, caractérisé en ce que la zone d'extrémité (6) courbée est de conformation cylindrique.

12. ensemble extenseur selon l'une quelconque des revendications précédentes, caractérisé en ce que le guide d'introduction (2) présente un diamètre intérieur correspondant au diamètre extérieur d'une pièce de guidage (4) pour le coulisseau (24).

13. Ensemble extenseur selon l'une quelconque des revendications précédentes, caractérisé en ce que cathéter endurétral introduit dans le guide d'introduction (2) vient en contact par son extrémité distale (13) avec l'extrémité proximale (14) de la pièce de guidage (4) et en ce que sa pointe arrondie (15) dépasse de l'extrémité avant du guide d'introduction (2).

14. Ensemble extenseur selon la revendication 13, caractérisé en ce que le diamètre extérieur du cathéter endurétral (12) correspond au diamètre intérieur du guide d'introduction (2).

15. Ensemble extenseur selon l'une quelconque des revendications 1 à 14, caractérisé en ce que le coulisseau (24) présente deux repères (25,26) en forme d'encoche à pouvoir de réflexion modifié par rapport au reste du coulisseau (24).

16. Ensemble extenseur selon la revendication 1 ou 15, caractérisé en ce que la longueur du coulisseau (24) correspond au moins à une fois et demie la longueur du guide d'introduction (2).

17. Ensemble extenseur selon l'une quelconque des revendications précédentes, caractérisé en ce que le diamètre extérieur du coulisseau (24) est inférieur aux diamètres intérieurs du cathéter endurétral (12) et le cas échéant de la pièce de guidage (4).

18. Ensemble extenseur selon l'une quelconque des revendications précédentes, caractérisé en ce que le guide d'introduction (2) est réalisé dans un matériau relativement rigide.

19. Ensemble extenseur selon l'une quelconque des revendications précédentes, caractérisé en ce que le cathéter endurétral (12) est réalisé dans un matériau à flexibilité élevée.

20. Ensemble extenseur selon l'une quelconque des revendications précédentes, caractérisé en ce que le coulisseau (24) est réalisé dans un matériau à flexibilité élevée mais relativement rigide axialement.

21. Ensemble extenseur selon la revendication 19 ou 20, caractérisé en ce que le matériau à flexibilité élevée est du polyéthylène.

22. Ensemble extenseur selon la revendication 19 ou 20, caractérisé en ce que le matériau à flexibilité élevée est du polyuréthane.
